(19) Europäisches Patentamt
European Patent Office
Office européen des brevets

(11) Veröffentlichungsnummer: **0 041 046**
**B1**

(12) # EUROPÄISCHE PATENTSCHRIFT

(45) Veröffentlichungstag der Patentschrift: **02.09.87**

(21) Anmeldenummer: **81810187.5**

(22) Anmeldetag: **18.05.81**

(51) Int. Cl.⁴: **C 07 D 235/10**, C 07 D 235/08, C 07 D 263/56, C 07 D 277/64, A 61 K 31/41

(54) **Benzazolderivate, Verfahren zu ihrer Herstellung, diese enthaltende pharmazeutische Präparate und ihre Verwendung.**

(30) Priorität: **22.05.80 CH 4016/80**

(43) Veröffentlichungstag der Anmeldung: **02.12.81 Patentblatt 81/48**

(45) Bekanntmachung des Hinweises auf die Patenterteilung: **02.09.87 Patentblatt 87/36**

(84) Benannte Vertragsstaaten: **BE CH DE FR IT LI LU NL**

(56) Entgegenhaltungen:
**DE-A-1 542 868**
**DE-A-1 545 874**
**DE-A-1 695 545**
**DE-A-2 140 496**
**US-A-3 849 431**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Gallay, Jean Jacques, Dr., Blumenrain 19, CH-4465 Magden (CH)**
Erfinder: **Schweizer, Ernst, Dr., Hollenweg 59, CH-4144 Arlesheim (CH)**

**EP 0 041 046 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung bezieht sich auf anthelminthisch wirkende Denzoxazol- und Denzthiazolderivate.

Aus DE-A-1 695 545 sind anthelminthisch wirkende Benzimidazolderivate bekannt, die sich von den Verbindungen der nachfolgenden Formel I dadurch unterscheiden, dass $X_3$ Stockstoff, $R_1$ unsubstituiertes Piperazin-1-yl und $R_3$ Heteroaryl ist. In US-A-3 849 431 sind ferner als anthelminthisch wirkende Verbindungen neben entsprechenden Benzimidazolen auch Benzoxazole und Benzthiazole beschrieben, die sich von den Verbindungen der nachfolgenden Formel I dadurch unterscheiden, dass sie an Stelle des Substituenten $R_1$-C(=$X_1$)-NH- die Gruppe SCN- aufweisen.

Die Erfindung betrifft nun eine Verbindung der Formel

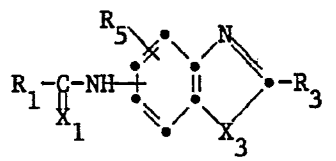

(I),

worin $R_1$ 4-$C_1$-$C_4$-Alkylpiperazin-1-yl, 4-$C_1$-$C_4$-Alkylpiperazin-4-oxid-1-yl, 4-(Hydroxy-$C_2$-$C_4$-alkyl)-piperazin-1-yl, worin die Hydroxygruppe in höherer als der $\alpha$-Stellung gebunden ist, oder eine gegebenenfalls mit einem $C_1$-$C_4$-Alkanol veresterte Carboxy-$C_1$-$C_4$-alkoxy- oder Carboxy-$C_1$-$C_4$-alkylthio-Gruppe, $X_1$ Sauerstoff oder Schwefel, $R_5$ Wasserstoff, $C_1$-$C_4$-Alkyl, $C_1$-$C_4$-Alkoxy, $C_1$-$C_4$-Alkylthio oder Halogen, $R_3$ $\alpha$-verzweigtes $C_3$-$C_4$-Alkyl oder $C_3$-$C_8$-Cycloalkyl und $X_3$ Sauerstoff oder Schwefel bedeuten, und Salze davon, Verfahren zu ihrer Herstellung, diese Verbindungen enthaltende pharmazeutische Präparate. Ferner betrifft sie die genanten Verbindungen zur Anwendung in einem Verfahren zur therapeutischen Behandlung des menschlichen oder tierischen Körpers.

$C_1$-$C_4$-Alkyl kann geradkettig oder verzweigt sowie in beliebiger Stellung gebunden sein und bedeutet im Falle von $R_1$ vorzugsweise geradkettiges, über ein sekundäres C-Atom gebundenes oder verzweigtes, vorzugsweise $\alpha$- oder $\beta$-verzweigtes, $C_1$-$C_4$-Alkyl und ansonsten vorzugsweise geradkettiges, über ein primäres oder sekundäres C-Atom gebundenes $C_1$-$C_4$-Alkyl. Über ein primäres C-Atom gebundenes Niederalkyl ist beispielsweise Methyl, Äthyl, 1-Propyl oder 1-Butyl. Über ein sekundäres C-Atom gebundenes geradkettiges Niederalkyl ist beispielsweise 2-Propyl oder 2-Butyl. Verzweigtes Niederalkyl ist beispielsweise Tertiärbutyl oder Isobutyl.

4-$C_1$-$C_4$-Alkyl-piperazin-1-yl ist beispielsweise 4-Methyl-, 4-Äthyl-, 4-Propyl-, 4-Isopropyl- oder 4-Butyl-, 4-Sekundärbutyl-, 4-Isobutyl- oder 4-Tertiärbutyl-piperazin-1-yl. Entsprechend bedeutet 4-$C_1$-$C_4$-Alkyl-4-oxido-piperazin-1-yl beispielsweise 4-Methyl-, 4-Äthyl-, 4-Propyl-, 4-Isopropyl- oder 4-Butyl-, 4-Sekundärbutyl-, 4-Isobutyl- oder 4-Tertiärbutyl-4-oxido-piperazin-1-yl. 4-(Hydroxy-$C_2$-$C_4$-alkyl)-piperazin-1-yl, worin die Hydroxygruppe in höherer als der $\alpha$-Stellung gebunden ist, bedeutet z. B. 4-(2-Hydroxyäthyl)- oder 4-(3-Hydroxypropyl)-piperazin-1-yl.

$C_1$-$C_4$-Alkoxy ist beispielsweise Methoxy, Äthoxy, Propyloxy, Isopropyloxy, Butyloxy, Sekundärbutyloxy, Isobutyloxy oder Tertiärbutyloxy.

Halogen ist beispielsweise Chlor, ferner Fluor oder Brom.

Salze von Verbindungen der Formel I sind beispielsweise deren pharmazeutisch verwendbare Salze, wie Säureadditionssalze oder im Falle saurer Verbindungen, z. B. solcher, in denen $R_1$ einen Carboxy-Rest enthält, deren Salze mit Basen, ferner im Falle von Verbindungen, in denen $R_1$ 4-substituiertes Piperazin-1-yl bedeutet, deren quaternäre Salze. Säureadditionssalze sind beispielsweise mineralsaure Salze, wie Halogenwasserstoffsäuresalze, z. B. Hydrochloride oder Hydrobromide, oder Schwefelsäuresalze, z. B. Hydrogensulfate oder Sulfate, Amidosulfonsäuresalze, wie gegebenenfalls N-substituierte Sulfaminsäuresalze, z. B. Sulfaminate oder N-Cyclohexylsulfaminate, oder organischen Sulfonate, z. B. Methan-, Äthan-, Benzol- oder p-Toluolsulfonate, ferner Carbonsäuresalze, wie Niederalkanoate, z. B. Acetate, oder Tatrate, Maleate, Fumarate, Maleinate und dergleichen. Salze mit Basen sind beispielsweise Metallsalze, wie Alkalimetallsalze, z. B. Natrium- oder Kaliumsalze, Erdalkalimetallsalze, z. B. Calcium-oder Magnesiumsalze, ferner Aluminium-, Zink- oder Kupfersalze, aber auch Ammoniumsalze mit Ammoniak oder organischen Aminen, z. B. mit Triäthylamin oder Äthanol-, Diäthanol- oder Triäthanolamin. Quaternäre Salze sind beispielsweise quaternäre Salze mit Niederalkylhalogeniden oder Niederalkylsulfonaten, z. B. 4,4-Dimethyl-, 4,4-Diäthyl- oder 4-Äthyl-4-methyl-halogenide oder Sulfonate, wie Metho- oder Äthosulfonate.

Die Erfindung betrifft vor allem Verbindungen der Formel

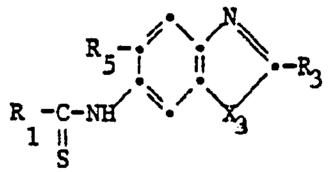

(Ia),

worin $R_1$ einerseits 1-(4-$C_1$-$C_4$-Alkyl)-piperazinyl, wie 1-(4-Methyl)-piperazinyl, 1-(4-$C_1$-$C_4$-Alkyl-4-oxido-piperazinyl, wie 1-(4-Methyl-4-oxid)-piperazinyl, oder 1-[4-(Hydroxy-$C_2$-$C_4$-alkyl)]-piperaziny l, worin die Hydroxygruppe in höherer als der α-Stellung gebunden ist, wie 1-[4-(2-Hydroxyäthyl)]-piperazinyl, oder andererseits eine gegebenenfalls mit einem $C_1$-$C_4$-Alkanol, wie Methanol oder Äthanol, veresterte Carboxy-$C_1$-$C_4$-alkoxy- oder Carboxy-$C_1$-$C_4$-alkylthio-Gruppe bedeutet, $R_5$ Wasserstoff, $C_1$-$C_4$-Alkyl, wie Methyl, $C_1$-$C_4$-Alkoxy, wie Methoxy, oder Halogen mit Atomnummer bis 35, wie Chlor, bedeutet, $X_3$ Sauerstoff oder Schwefel darstellt und $R_3$ für α-verzweigtes $C_3$-$C_4$-Alkyl, wie Tertiärbutyl oder Isopropyl steht, und ihre Salze, insbesondere pharmazeutisch verwendbare Salze.

Die Erfindung betrifft insbesondere Verbindungen der Formel Ia, worin $R_1$ einerseits 1-(4-$C_1$-$C_4$-Alkyl)-piperazinyl, wie 1-(4-Methyl)-piperazinyl, 1-(4-$C_1$-$C_4$-Alkyl-4-oxid)-piperazinyl, wie 1-(4-Methyl-4-oxido)-piperazinyl, oder 1-[4-(Hydroxy-$C_2$-$C_4$-alkyl)]-piperazinyl, worin die Hydroxygruppe in höherer als der α-Stellung gebunden ist, wie 1-[4-(2-Hydroxyäthyl)]-piperazinyl, oder andererseits eine Carboxy oder $C_1$-$C_4$-Alkoxycarbonyl-$C_1$-$C_4$-alkylthiogruppe, z. B. eine Methoxycarbonyl- oder Äthoxycarbonyl-$C_1$-$C_4$-alkylthiogruppe bedeutet und worin $R_5$ Wasserstoff, $C_1$-$C_4$-Alkyl, wie Methyl, $C_1$-$C_4$-Alkoxy, wie Methoxy, oder Halogen mit Atomnummer bis 35, wie Chlor, bedeutet, $X_3$ Thio darstellt und $R_3$ α-verzweigtes $C_3$-$C_4$-Alkyl, wie Tertiärbutyl oder Isopropyl, darstellt, und ihre Salze.

Die Erfindung betrifft in allererster Linie Verbindungen der Formel Ia, worin $R_1$ einerseits 1-(4-$C_1$-$C_4$-Alkyl)-piperazinyl, wie 1-(4-Methyl)-piperazinyl, bedeutet oder andererseits ω-Carboxy-$C_1$-$C_4$-alkylthio, wie 2-Carboxyäthylthio, darstellt, und worin $R_5$ Wasserstoff, $C_1$-$C_4$-Alkoxy oder $C_1$-$C_4$-Alkyl, wie Methoxy oder Methyl, bedeutet, $X_3$ Thio ist und $R_3$ α-verzweigtes $C_3$-$C_4$-Alkyl, wie Tertiärbutyl, bedeutet, und ihre Salze.

Die Erfindung betrifft namentlich die in den Beispielen genannten Verbindungen der Formel I und ihre Salze.

Die Verbindungen der Formel I und ihre Salze werden nach an sich bekannten Verfahren hergestellt, indem man

a) Verbindungen der Formel II und III

$R_1$—$Y_1$ (II) und (III)

$R_1$—$Y_1$ (II) und 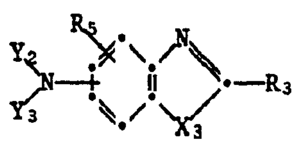 (III) ,

worin $Y_1$ Wasserstoff bedeutet und $Y_2$ und $Y_3$ gemeinsam die Gruppe der Formel $=C=X_1$ bedeuten, oder einer der Reste $Y_1$ und $Y_2$ die Gruppe der Formel -C(=$X_1$)-$Y_4$ bedeutet, wobei $Y_4$ für einen abspaltbaren Rest steht, und der andere Rest und $Y_3$ Wasserstoff darstellen oder deren Salze miteinander kondensiert, oder

b) in einer Verbindung der Formel IV

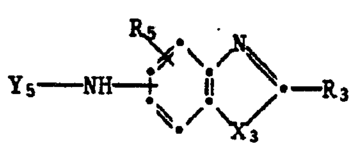

(IV),

worin $Y_5$ einen in die Gruppe der Formel $R_1$-C(=$X_1$)- überführbaren Rest bedeutet, oder in einem Salz davon, $Y_5$ in die Gruppe der Formel $R_1$-C(=$X_1$)- überführt, oder

c) eine Verbindung der Formel

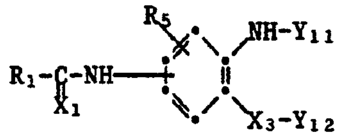

(V),

worin einer der Reste $Y_{11}$ und $Y1_{12}$ die Gruppe der Formel -C(=$Y_{13}$)-$R_3$ und der andere einen abspaltbaren Rest bedeutet und $Y_{13}$ für gegebenenfalls funktionell abgewandeltes Oxo steht, oder ein Salz davon cyclisiert und gewünschtenfalls eine erfindungsgemäss erhältliche Verbindung in eine andere Verbindung der Formel I umwandelt und/oder ein erhältliches Salz in die freie Verbindung oder in ein anderes Salz überführt.

Verfahren a):

Der abspaltbare Rest $Y_4$ ist beispielsweise gegebenenfalls verestertes oder veräthertes Hydroxy, gegebenenfalls veräthertes Mercapto oder quaternäres Ammonio. Verestertes Hydroxy ist beispielsweise mit einer organischen Carbonsäure verestertes Hydroxy, wie Niederalkanoyloxy, wie Acetoxy, oder eine Benzoyloxygruppe, vorzugsweise aber reaktionsfähiges verestertes Hydroxy, wie Halogen, z. B. Chlor oder Brom. Veräthertes Hydroxy ist beispielsweise Niederalkoxy oder eine Benzyloxy- oder Phenoxygruppe. Entsprechend ist veräthertes Mercapto beispielsweise Niederalkylthio oder eine Benzylthio- oder Phenylthiogruppe. Quaternäres Ammonio ist beispielweise Triniederalkylammonio, z. B. Trimethyl- oder Triäthylammonio, oder Pyridinio. Ausgehend von Ausgangsstoffen der Formel II, worin $R_1$ einen in 4-Stellung substituierten 1-Piperazinylrest bedeutet, kommt als abspaltbarer Rest auch primäres, sekundäres oder tertiäres Amino in Betracht, beispielsweise Amino, N-Mono- oder N,N-Diniederalkylamino, z. B. Methyl- oder Äthylamino, oder Dimethyl- oder Diäthylamino, N,N-Alkylenamino, wie Piperidino, Pyrrolidino, Morpholino, Thiomorpholino oder eine Anilino- oder Diphenylaminogruppe. Dabei können Benzoyloxy-, Benzyloxy-, Phenoxy-, Phenylthio-, Pyridino-, Anilino- und Diphenylaminogruppen, ebenso kann der Phenylteil von Benzyloxy- und Benzylthiogruppen auch z. B. durch Niederalkyl, Niederalkoxy, Halogen und/oder Nitro, substituiert sein.

Salze von Ausgangsstoffen der Formeln II und III sind beispielsweise Metallsalze von Verbindungen der Formel II, worin $R_1$ eine Carboxygruppe HOOC- enthält, beispielsweise Alkalimetall- oder Erdalkalimetallsalze, z. B. die Natrium- oder Kaliumsalze derselben, ferner Säureadditionssalze von Verbindungen der Formel II, worin $R_1$ in 4-Stellung substituiertes 1-Piperazinyl und $Y_1$ Wasserstoff bedeutet, bzw. von Verbindungen der Formel III, worin $Y_2$ einen Rest -C(=$X_1$)-$Y_4$ oder Wasserstoff, $Y_3$ Wasserstoff und $Y_4$ reaktionsfähiges verestertes Hydroxy darstellt, beispielsweise deren Mineralsäureadditionssalze, wie Hydrohalogenide, z. B. Hydrochloride oder Hydrobromide, oder Schwefelsäureadditionssalze, z. B. Hydrogensulfate.

Die Umsetzung wird in üblicher, insbesondere in der aus der Literatur für analoge Reaktionen bekannten Weise durchgeführt, vorzugsweise in einem Lösungsmittel, beispielsweise einem Niederalkanol, erforderlichenfalls in Gegenwart eines Kondensationsmittels, bei der Umsetzung von Verbindungen der Formeln II und III, worin einer der Reste $Y_1$ und $Y_2$ eine Gruppe -C(=$X_1$)-$Y_4$ und $Y_4$ reaktionsfähiges verestertes Hydroxy bedeutet, vorzugsweise eines basischen Kondensationsmittels, bei der Umsetzung von Verbindungen der Formeln II und III, worin einer der Reste $Y_1$ und $Y_2$ eine Gruppe -C(=$X_1$)-$Y_4$ und $Y_4$ eine gegebenenfalls verätherte Mercaptogruppe oder eine verätherte Hydroxygruppe bedeutet, unter, beispielsweise destillativer oder azeotrop-destillativer, Entfernung des abgespaltenen Schwefelwasserstoffes, Mercaptans oder Alkohols, und/oder bei erhöhter Temperatur, z. B. bei Siedetemperatur. Basische Kondensationsmittel sind zur Bindung von freigesetzter Halogenwasserstoffsäure geeignete Basen, wie anorganische Basen, z. B. Natrium-, Kalium- oder Calciumhydroxid, -carbonat oder -hydrogencarbonat, oder organische Stickstoffbasen, z. B. Triäthylamin oder Pyridin.

Die Ausgangsstoffe der Formeln II und III sind zum Teil bekannt. Neue Verbindungen der Formeln II und III können in Analogie zur Bildungsweise der bekannten Ausgangsstoffe hergestellt werden.

Verbindungen der Formeln II bzw. III, worin $Y_1$ bzw. $Y_2$ eine Gruppe -C(=$X_1$)-$Y_4$ bedeutet, können z. B. hergestellt werden, indem man eine Verbindung der Formel II bzw. III, worin $Y_1$ bzw. $Y_2$ und/oder $Y_3$ Wasserstoff bedeutet, in üblicher Weise mit einer Verbindung der Formel $Y'_4$-C(=$X_1$)-$Y'_4$ (IIa), worin die beiden Reste $Y'_4$ unabhängig voneinander reaktionsfähig verestertes Hydroxy, wie Halogen, veräthertes Hydroxy oder veräthertes Mercapto $Y_4$ bedeutet, umsetzt, beispielsweise mit Phosgen, Thiophosgen, einem Halogenameisensäure-, Halogenthioameisensäure- oder Halogendithioameisensäureester oder mit einem N,N-disubstituierten, wie N,N-diniederalkylierten, Carbamoylhalogenid.

Verbindungen der formel III, worin $Y_2$ und $Y_3$ gemeinsam für eine Gruppe =C=$X_1$ stehen, können z. B. erhalten werden durch Umsetzung einer Verbindung der Formel III, worin $Y_2$ und $Y_3$ Wasserstoff bedeuten, mit einer Verbindung der Formel $Y'_4$-C(=$X_1$)-$Y'_4$ (IIa), worin die beiden Reste $Y'_4$ unabhängig voneinander reaktionsfähig verestertes Hydroxy, veräthertes Hydroxy oder veräthertes Mercapto bedeuten, wie mit Phosgen, Thiophosgen, einem Halogenameisensäure-, Halogenthioameisensäure- oder Halogendithioameisensäureester, oder einem N,N-disubstituierten, wie N,N-diniederalkylierten Carbamoylhalogenid, in Gegenwart eines basischem Kondensationsmittels, wie einer anorganischen Base, von Pyridin oder einem Triniederalkylamin, oder eines Carbodiimides, mit einem tertiären Amin und Schwefelkohlenstoff, mit einem gegebenenfalls substituierten Benzoylisothiocyanat oder mit einem

Alkalimetall- oder Ammoniumrhodanid, und jeweils thermische Zersetzung der gebildeten Thioharnstoffverbindung, oder durch Umsetzung mit Chlorwasserstoff und Ammoniumcyanat bzw. Ammoniumthiocyanat. Dabei werden primär die als Ausgangsstoffe erwähnten Verbindungen der Formel III, worin $Y_2$ eine Gruppe $-C(=X_1)-Y_4$ bedeutet, gebildet, die entweder durch Umsetzung mit einer Verbindung der Formel II, worin $Y_1$ Wasserstoff ist, abgefangen werden können oder in Abwesenheit derselben zu Isocyanaten bzw. Isothiocyanaten der Formel III weiterreagieren. Ausgangsstoffe der Formel III, worin $Y_2$ eine Gruppe $-C(=X_1)-Y_4$ und $Y_3$ Wasserstoff bedeutet, werden dementsprechend vorzugsweise unter den Reaktionsbedingungen ihrer Weiterverarbeitung in situ gebildet und ohne Isolierung weiterumgesetzt.

Verfahren b):

In eine Gruppe der Formel $R_1-C(=X_1)-$ überführbare Reste sind beispielsweise solche, die durch Solvolyse, d.h. Umsetzung mit Wasser, (Hydrolyse), Schwefelwasserstoff bzw. einem seiner Salze oder einem Alkohol (Alkoholyse) oder durch Reduktion bzw. Oxidation in eine Gruppe der Formel $R_1-C(=X_1)-$ überführt werden können.

Salze von Verbindungen der Formel IV sind beispielsweise Säureadditionssalze, wie Hydrochloride oder Sulfonsäuresalze derselben.

Durch Solvolyse in Gruppen der Formel $R_1-C(=X_1)-$ überführbare Reste sind beispielsweise solche der Formeln $Y_6-C(=X_1)-$, $R_1-C(Y_7Y_8)-$ oder $Y_6-C(Y_7Y_8)-$, worin $Y_7$ und $Y_8$ unabhängig voneinander veresterte oder mit einem einwertigen Alkohol verätherte Hydroxygruppen oder mit einem einwertigen Mercaptan verätherte Mercaptogruppen oder gemeinsam mit einem zweiwertigen Alkohol verätherte Hydroxygruppen bzw. mit einem zweiwertigen Mercaptan verätherte Mercaptogruppen oder eine gegebenenfalls in Salzform vorliegende Iminogruppe darstellen und $Y_6$ eine in 4-Stellung durch einen solvolytisch abspaltbaren Rest substituierte 1-Piperazinogruppe, oder eine Gruppe $Y_9$-alk-$X_2$- darstellt, worin $Y_9$ eine veresterte oder mit einem Silanol oder einem cyclischen 2-Hydroxyäther bzw. 2- oder 4-Hydroxythioäther verätherte oder mit einer Carbonsäure veresterte Hydroxymethylgruppe bedeutet oder für von verestertem Carboxy verschiedenes funktionell abgewandeltes Carboxy der Formel $-C(=O)-Y_6$ steht.

Verestertes Hydroxy ist beispielsweise mit einer Halogenwasserstoffsäure oder einer organischen Carbonsäure verestertes Hydroxy, wie Halogen, z. B. Chlor oder Brom, Niederalkanoyloxy, z. B. Acetoxy oder Pivaloyloxy, oder gegebenenfalls substituiertes Benzoyloxy. Mit einem einwertigen Alkohol veräthertes Hydroxy ist beispielsweise Niederalkoxy, z. B. Methoxy oder Äthoxy. Ebenso ist mit einem einwertigen Mercaptan veräthertes Mercapto beispielsweise Niederalkylthio, z. B. Methylthio oder Äthylthio. Mit einem zweiwertigen Alkohol verätherte Hydroxygruppen sind beispielsweise Niederalkylendioxy, z. B. Äthylendioxy oder Propylendioxy, und mit einem zweiwertigen Mercaptan verätherte Mercaptogruppen sind beispielsweise Niederalkylendithio, z. B. Äthylendithio oder Propylendithio.

Gegebenenfalls in Salzform vorliegende Iminogruppen sind beispielsweise gegebenenfalls als Hydrohalogenid, wie Hydrochlorid oder Hydrobromid, Sulfonsäuresalz, z. B. Methansulfonat oder p-Toluolsulfonat, oder Schwefelsäuremonoestersalz, z. B. Methosulfat, vorliegendes Imino. Solvolytisch abspaltbare Reste in 4-Stellung einer Piperazinogruppe sind beispielsweise die einer organischen Carbonsäure oder einem Halbester oder Monohalogenid der Kohlensäure abgeleitete Acylreste, wie Niederalkanoyl, z. B. Acetyl, oder gegebenenfalls substituiertes Benzoyl, Niederalkoxy- oder gegebenenfalls substituierte Benzyloxy- bzw. Phenyloxycarbonylgruppen oder Halogencarbonyl.

Mit einem Silanol verätherte Hydroxymethylgruppen sind beispielsweise Triniederalkylsilyloxymethylgruppen, wie Trimethylsilyloxymethyl. Mit einem cyclischen 2-Hydroxyäther veräthertes Hydroxy ist beispielsweise 2-Tetrahydropyranyloxymethyl und mit einem cyclischen 2- oder 4-Hydroxythioäther beispielsweise 2-Tetrahydrothiopyranyloxymethyl oder 4-Dihydrothiopyranyloxymethyl.

Mit einer Carbonsäure verestertes Hydroxymethyl ist beispielsweise mit einer organischen Carbonsäure oder einem Halbester oder Monohalogenid der Kohlensäure verestertes Hydroxymethyl, wie Niederalkanoyloxymethyl, z. B. Acetoxymethyl, gegebenenfalls substituiertes Benzoyloxymethyl, Niederalkoxycarbonyloxymethyl, gegebenenfalls substituiertes Benzyloxy- bzw. Phenoxycarbonyloxymethyl oder Chlorcarbonyloxymethyl. Von verestertem Carboxy verschiedene funktionell abgewandelte Carboxygruppen $-C(=O)-Y_6$ sind vorzugsweise Cyano, gegebenenfalls in Salzform, z. B. als Hydrohalogenid, Methan- oder p-Toluolsulfonat oder Methosulfat, vorliegende Iminoäther- oder Iminoestergruppierungen oder Orthoester- bzw. Orthoanhydridgruppierungen, wie Triniederalkoxymethyl oder Trihalogen- z. B. Trichlormethyl, ferner amidiertes oder anhydriertes Carboxy, wie ggf. substituiertes Carbamoyl oder Halogencarbonyl.

Durch Hydrolyse der genannten Gruppen erhält man beispielsweise ausgehend von Resten $Y_5$ der Formeln $Y_6-C(Y_{7-8})$ $R_1-C(Y_7Y_8)-$ und $Y_6-C(=O)$-Gruppen der Formel $R_1-C(=O)$ bzw. ausgehend von Resten der Formel $Y_6-C(=S)-$ bzw. $Y_6-C(Y_7Y_8)$, worin $Y_7$ und $Y_8$ veräthertes Mercapto darstellen, Gruppen der Formel $R_1-C(=S)-$, worin $R_1$ 4-unsubstituiertes 1-Piperazinyl, Carboxyl-$C_1$-$C_4$-alkoxy oder -alkylthio ist, ferner ausgehend von Gruppen $Y_6-C(=X_1)-$, worin $Y_6$ eine Gruppe $Y_9$-$C_1$-$C_4$-alkoxy oder $Y_9$-$C_1$-$C_4$-alkylthio und $Y_9$ eine Iminoäther- oder Orthoestergruppe ist, solche der Formel $R_1-C(=X_1)-$, worin $R_1$ durch $C_1$-$C_4$-Alkanol verestertes Carboxy-$C_1$-$C_4$-alkoxy oder Carboxy-$C_1$-$C_4$-alkylthio ist.

Die Hydrolyse erfolgt in üblicher Weise, beispielsweise in Gegenwart eines Hydrolysemittels, erforderlichenfalls in einem mit Wasser mischbaren organischen Lösungsmittel, bei erhöhter oder erniedrigter Temperatur, z. B. im Temperaturbereich von etwa 0° bis 120°C, und/oder unter Inertgas, wie Stickstoff. Als Hydrolysemittel kommen beispielsweise saure Hydrolysemittel, ausgehend von Verbindungen der Formel IV,

worin $Y_5$ einen in 4-Stellung durch eine hydrolytisch abspaltbare Gruppe substituierten 1-Piperazinylrest oder mit einer Carbonsäure veresterte Hydroxymethylgruppe bzw. eine funktionell abgewandelte Carboxygruppe $Y_9$ aufweist, auch basische Hydrolysemittel in Betracht. Saure Hydrolysemittel sind beispielsweise Mineralsäuren, wie Halogenwasserstoffsäuren, z. B. Salzsäure, oder deren saure Salze, z. B. Ammoniumchlorid, oder Schwefelsäure bzw. Hydrogensulfate, ferner organische Carbonsäure, wie Niederalkansäuren, z. B. Essigsäure. Basische Hydrolysemittel sind beispielsweise Hydroxide oder Carbonate von Alkali- oder Erdalkalimetallen, z. B. Natrium-, Kalium- oder Calciumhydroxid, Natriumcarbonat oder Kaliumcarbonat. Mit Wasser mischbare organische Lösungsmittel sind beispielsweise Alkohole, wie Niederalkanole, z. B. Methanol oder Äthanol, Diniederalkylketone, z. B. Aceton, cyclische Äther, wie Dioxan oder Tetrahydrofuran, Dimethylformamid oder Dimethylsulfoxid.

Durch Umsetzung mit Schwefelwasserstoff oder einem seiner Salze, wie einem Metall oder Ammomiumsulfid, z. B. mit Kalium-, Natrium- oder Ammoniumsulfid, kann man beispielsweise Gruppen der Formel $R_1$-C($Y_7Y_8$)- in solche der Formel $R_1$-C(=S)- umwandeln.

Die Umsetzung mit Schwefelwasserstoff bzw. einem seiner Salze erfolgt in üblicher Weise, beispielsweise in einem Lösungsmittel und erforderlichenfalls in Gegenwart eines üblichen Katalyse- oder Kondensationsmittels, unter Kühlen oder Erwärmen - z. B. im Temperaturbereich von etwa 0° bis 120°C - und/oder unter Inertgas, wie Stickstoff. Als Lösungsmittel kommen beispielsweise polare Lösungsmittel in Betracht, wie Wasser, Alkohole, z. B. Methanol oder Äthanol, cyclische Äther, wie Tetrahydrofuran, Dimethylformamid oder Dimethylsulfoxid oder Gemische derselben.

Durch Alkoholyse kann man beispielsweise Gruppen der Formel $Y_6$-C(=$X_1$)-, worin $Y_6$ eine Gruppe $Y_9$-$C_1$-$C_4$-alkoxy- oder $Y_9$-$C_1$-$C_4$-alkylthio und $Y_9$ anhydridisiertes Carboxy oder mit einer organischen Carbonsäure verestertes Hydroxymethyl ist, in verestertes Carboxy-$C_1$-$C_4$-alkoxy oder verestertes Carboxy-$C_1$-$C_4$-alkylthio oder Hydroxymethyl überführen.

Die Alkoholyse erfolgt in üblicher Weise beispielsweise in Gegenwart eines sauren oder basischen Mittels und erforderlichenfalls in einem Lösungsmittel, unter Kühlen oder Erwärmen - z. B. im Temperaturbereich von etwa 0° bis 120°C - und/oder unter Inertgas. Saure Mittel sind beispielsweise, Mineralsäuren, wie Halogenwasserstoffsäuren, z. B. Chlor- oder Bromwasserstoff, oder Schwefelsäure. Basische Mittel sind beispielsweise Alkalimetallhydroxide, z. B. Natrium- oder Kaliumhydroxid, oder Alkalimetallalkoholate, wie das Natrium- oder Kaliumalkoholat des verwendeten Alkohols. Lösungsmittel sind beispielsweise mit dem zu verwendenden Alkohol mischbare Lösungsmittel oder auch ein Überschuss desselben.

Durch Oxidation kann man beispielsweise eine Gruppe $Y_5$ der Formel O=CH-$C_1$-$C_4$-alkoxy- oder -thio-C(=$X_1$)- in eine Gruppe $R_1$ der Formel HOOC-$C_1$-$C_4$-alkoxy- oder -thio-C(=$X_1$)- überführen. Als Oxidationsmittel kommen dabei beispielsweise oxidierende Schwermetallverbindungen in Betracht, wie Chrom-VI-, -Mangan-IV- und Mangan-VII- Verbindungen, z. B. Chromtrioxid, Chromsäure bzw. Chromsäuresalze, Mangandioxid oder Kaliumpermangant, ferner Sauerstoff, erforderlichenfalls in Gegenwart eines Lösungsmittels, z. B. von Wasser, einer Carbonsäure, wie Essigsäure, eines Ketons, wie Aceton, oder von Gemischen, auch wasserhaltigen Gemischen, derselben, unter Kühlen oder Erwärmen - z. B. im Temperaturbereich von etwa - 20° bis + 100°C -, im einem geschlossenen Gefäss und/oder unter Inertgas, wie Stickstoff.

Bei den vorstehend erwähnten Oxidationen kann die Formylgruppe in einer Verbindung der Formel IV, worin $Y_5$ eine Gruppe O=HC-$C_1$-$C_4$-alkoxy- oder -thio-C(=$X_1$)- darstellt, auch in situ, beispielsweise durch Oxidation einer gegebenenfalls veresterten Hydroxymethylgruppe, wie einer gegebenenfalls mit einer Halogenwasserstoffsäure oder einer Carbonsäure veresterten Hydroxymethylgruppe, z. B. aus Hydroxymethyl, Chlor- oder Brommethyl, Niederalkanoyloxymethyl oder Niederalkoxycarbonyloxymethyl gebildet oder aus einer funktionell abgewandelten Formylgruppe, wie einer acetalisierten, thioacetalisierten oder acylalisierten Formylgruppe, z. B. aus Diniederalkoxy- bzw. Niederalkylendioxymethyl, Diniederalkylthio- bzw. Niederalkylendithio, Dichlormethyl oder Diniederalkanoyloxymethyl, oder aus einer gegebenenfalls substituierten Iminomethylgruppe, z. B. Iminomethyl oder Anilomethyl, in Freiheit gesetzt werden, beispielsweise durch Hydrolyse.

Neue Ausgangsstoffe der Formel IV können in Analogie zur Bildungsweise bekannter hergestellt werden.

So erhält man Verbindungen der Formel IV, in denen $Y_5$ eine Gruppe der Formel $Y_6$-C(=$X_1$)- oder $Y_{10}$-C(=$X_1$)- bedeutet, beispielsweise indem man eine Verbindung der Formel $Y_6$-H (IVa) bzw. $Y_{10}$-H (IVb) mit einer Verbindung der Formel

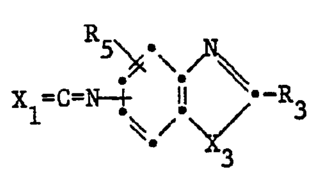

(IVc)

oder eine Verbindung der Formel $Y_6$-C(=$X_1$)-Hal (IVd) bzw. $Y_{10}$-C(=$X_1$)-Hal (IVe), worin Hal Halogen, wie Chlor, ist, mit einer Verbindung der Formel

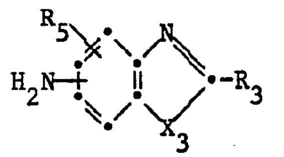

(IVf)

umsetzt, vorzugsweise in der für die Umsetzungvon Verbindungen der formeln II und III angegebenen Weise.

Verbindungen der Formel IV, worin $Y_5$ eine Gruppe $R_1$-C($Y_7Y_8$)- oder $Y_6$-C($Y_7Y_8$)-bedeutet, erhält man beispielsweise indem man eine Verbindung der Formel IVd bzw. IVe in üblicher Weise zunächst mit einem Orthoameisensäurederivat der Formel H-C($Y_7Y_7Y_8$) IVg und anschliessend mit einer Verbindung der Formel IVf kondensiert.

Verfahren c):

Abspaltbare Reste in Verbindungen der Formel (V) sind beispielsweise Gruppen der Formel -C(=$Y_{13}$)-$R_3$, Wasserstoff, Acyl oder gegebenenfalls in Salzform vorliegendes, an Thio $X_3$ gebundenes Sulfo. Acyl ist dabei insbesondere solches der Formel $R_3$-C(=O)-. Funktionell abgewandeltes Oxo ist beispielsweise Thioxo oder Imino.

Salze von Verbindungen der Formel V sind beispielsweise deren Säureadditionssalze, wie Hydrohalogenide, z. B. Hydrochloride, oder Alkalimetallsalze von Verbindungen der Formel V, worin -$X_3$-$Y_{12}$ Sulfo, Hydroxy oder Mercapto ist, wie deren Natrium- oder Kaliumsalze.

Die Cyclisierung erfolgt in üblicher Weise, erforderlichenfalls in Gegenwart eines Kondensationsmittels und/oder eines Lösungsmittels, wenn nötig unter Kühlen oder Erwärmen - z. B. im Temperaturbereich von etwa 0° bis 130° C-, in einem geschlossenen Gefäss und/oder unter Inertgas, wie Stickstoff. Kondensationsmittel sind beispielsweise saure oder basische Kondensationsmittel. Saure Kondensationsmittel, deren Verwendung insbesondere ausgehend von Verbindungen der Formel V, worin $Y_{12}$ Acyl bedeutet, angezeigt ist, sind beispielsweise Mineralsäuren bzw. deren sauren Salze und sauren Anhydride, wie Halogenwasserstoffsäure, z. B. Chlorwasserstoff, Schwefelsäure oder Hydrogensulfate, z. B. Kalium- oder Ammoniumhydrogensulfat, Phosphorsäure bzw. deren saure Anhydride, wie Polyphosphorsäure oder Borsäure, ferner Sulfonsäuren, wie p-Toluolsulfonsäure, oder Lewissäuren, wie Bortrifluorid oder Antimonpentachlorid. Statt in Gegenwart eines sauren Kondensationsmittels zu arbeiten, kann man die zu cylisierende Verbindung auch als Säureadditionssalz einsetzen. Basische Kondensationsmittel sind beispielsweise Alkalimetall- oder Erdalkalimetallhydroxide, wie Natrium-, Kalium- oder Calciumhydroxid, Alkalimetallcarbonate, z. B. Natrium- oder Kaliumcarbonat, oder organische Stickstoffbasen, vorzugsweise tertiäre Amine, wie Pyridin oder Triniederalkylamino, z. B. Triäthylamin. Statt in Gegenwart einer Base zu arbeiten, kann man eine Verbindung der Formel V, worin -$X_3$-$Y_{12}$ Hydroxy oder Mercapto bedeutet, auch als Alkalimetallsalze einsetzen. Als Lösungsmittel kommen beispielsweise aromatische oder araliphatische Kohlenwasserstoffe, z. B. Benzol, Toluol, Xylole, Halogenkohlenwasserstoffe, z. B. Di-, Tri- und Tetrachlormethan, aliphatische oder cyclische Äther, z. B. Diäthylether, Dioxan, Tetrahydrofuran, Dimethylformamid oder Dimethylsulfoxid, ferner Wasser und wasserhaltige Lösungmittel der genannten Art in Betracht.

Neue Ausgangsstoffe der Formel V können in analoger Weise wie bekannte hergestellt werden.

So erhält man Verbindungen der formel V, worin $Y_{11}$ eine Gruppe der Formel -C(=$Y_{13}$)-$R_3$ und $Y_{12}$ Wasserstoff oder ebenfalls eine Gruppe der Formel -C(=$Y_{13}$)-$R_3$ bedeutet, beispielsweise, indem man eine Verbindung der Formel Va

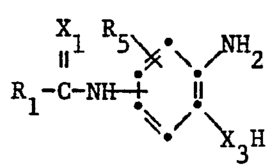

(Va)

mit einer Verbindung der Formel Z-C(=$Y_{13}$)-$R_3$ (Vb), worin Z Hydroxy und $Y_{13}$ Oxo, oder Z veräthertes Hydroxy und $Y_{13}$ Oxo, der Imino oder reaktionsfähiges verestertes Hydroxy, z. B. Halogen und $Y_{13}$ Oxo, Thioxo oder Imino oder Z eine Gruppe der Formel -O-C(=O)-$R_3$ und $Y_{13}$ Oxo bedeutet, oder Z und $Y_{13}$ gemeinsam für Nitrilo stehen, oder einem Salz, z. B. einem Immoniumhydrohalogenid eimer Verbindung der Formel Vb, worin $Y_{13}$ Imino ist, umsetzt. Dabei arbeitet man in der üblichen Weise, bei der Umsetzung mit Verbindungen der Formel Vb, worin Z reaktionsfähiges verestertes Hydroxy oder eine Gruppe -O-C(=$Y_{13}$)-$R_3$ bedeutet, in Gegenwart eines basischen Kondensationsmittels, bei der Umsetzung mit Verbindungen der Formel Vb, worin Z und $Y_{13}$ gemeinsam für Nitrilo stehen, eines sauren Kondensationsmittels und bei der Umsetzung mit Verbindungen der Formel Vb, worin Z Hydroxy und $Y_{13}$ Oxo ist, eines wasserbindenden Mittels. Basische Kondensationsmittel sind beispielsweise Alkalimetall- und Erdalkalimetallhydroxide, z. B. Natrium-, Kali - oder Calciumhydroxid, Alkalimetallcarbonate, z. B. Natrium- oder Kaliumcarbonat, Alkalimetall-, Erdalkalimetall- und Ammoniumsalze von Säuren der Formel HO-C(=O)-$R_3$, wie deren Natrium-, Kalium-, Ammonium- oder Magnesiumsalze, oder tertiäre organische Stickstoffbasen, wie Pyridin oder Triniederalkylamine, wie

Triäthylamin. Säure Kondensationsmittel sind beispielsweise Halogenwasserstoffsäuren, wie Chlor- oder Brom wasserstoff. Wasserbindende Mittel sind beispielsweise Carbodiimide, wie Dicyclohexylcarbodiimid.

Die als Ausgangsmaterial dienenden Verbindungen der Formel Va können z. B. erhalten werden, indem man in einer entsprechenden Nitroverbindung (statt $-NH_2$ Nitro enthaltend) oder einem Disulfid (statt $-X_3H$ die Gruppe $-S-S-Ph(NO_2)-NH-C(=X_1)-R_1$ enthaltend) die Nitrogruppen und gegebenenfalls die Disulfidbrücke reduziert, z. B. durch Umsetzung mit Wasserstoff in Gegenwart eines Hydrierungskatalysators, z. B. von Palladium auf Kohle, mit reduzierenden Metallen oder Metallverbindungen, z. B. mit Eisen, Zink, Zinn oder Zinn-II-chlorid, oder, zur Reduktion einer Disulfidbrücke, mit Natriumborhydrid bzw., zur Reduktion von Nitro, sulfiertem Natriumborhydrid.

Verbindungen der Formel V, worin $Y_{11}$ Wasserstoff und $Y_{12}$ eine Gruppe $-C(=Y_{13})-R_3$ bedeutet, können z. B. hergestellt werden, indem man eine Verbindung der Formel

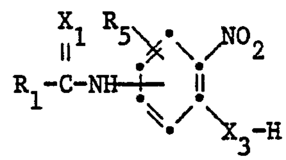

(Vc)

mit einer Verbindung der Formel $Z-C(=Y_{13})-R_3$ (Vb), worin Z Hydroxy und $Y_{13}$ Oxo, oder Z veräthertes Hydroxy und $Y_{13}$ Oxo oder Imino, oder Z reaktionsfähiges verestertes Hydroxy, z. B. Halogen, und $Y_{13}$ Oxo, Thioxo oder Imino, oder Z eine Gruppe der Formel $-O-C(=O)-R_3$ und $Y_{13}$ Oxo bedeutet, oder Z und $Y_{13}$ gemeinsam für Nitrilo stehen oder einem Salz, z. B. einem Immoniumhydrohalogenid einer Verbindung der Formel Vb, worin $Y_{13}$ Imino ist, umsetzt und die Nitrogruppe reduziert, z. B. mittels sulfierten Natriumborhydrides oder reduzierender Metalle oder Metallverbindungen, z. B. mittels Eisen, Zink, Zinn oder Zinn-II-chlorid. Bei der Umsetzung mit Verbindungen der Formel Vb arbeitet man wie oben angegeben.

Die dafür als Ausgangsstoffe dienenden Verbindungen der Formel Vc können z. B. hergestellt werden, indem man in einer Verbindung der Formel

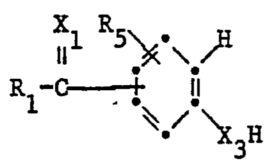

(Vd)

die $X_3H$-Gruppe schützt, z. B. durch Benzylierung, Carbobenzoxylierung oder Oxidation einer Mercaptoverbindung ($X_3H$ = SH) zum Disulfid, dann nitriert und anschliessend, z. B. reduktiv, die Gruppe $-X_3H$, freisetzt.

Verbindungen der Formel V, worin $Y_{11}$ eine Gruppe der Formel $-C(=Y_{13})-R_3$, $X_3$ Thio und $Y_{12}$ gegebenenfalls in Salzform vorliegendes Sulfo ist, können ferner hergestellt werden, indem man eine Verbindung der Formel

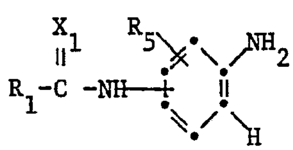

(Ve)

in üblicher Weise durch Umsetzung mit einem Thiosulfat, z. B. mit Natriumthiosulfat in das Bunte-Salze (Formel V; $Y_{11}$ = H; $-X_3-Y_{12}$ = $-S-SO_3\theta$) überführt und dieses in der angegebenen Weise mit einer Verbindung der Formel Vb weiterumsetzt.

Erfindungsgemäss erhältliche Verbindungen können in andere Verbindungen der Formel I umgewandelt werden.

So kann man beispielsweise in einer Verbindung der Formel I, worin $R_1$ verestertes Carboxy erthält, dieses in üblicher Weise zu Carboxy hydrolysieren, beispielsweise in Gegenwart eines sauren oder basischen Kondensationsmittels, erforderlichenfalls in Gegenwart eines Lösungsmittels, unter Kühlen oder Erwärmen - z. B. im Temperaturbereich von etwa 0 bis 120° C - und/oder unter Inertgas, wie Stickstoff. Saure Hydrolysemittel sind beispielsweise Protonensäure, wie Mineralsäuren, z. B. Chlor oder Bromwasserstoffsäure, Schwefelsäure oder Phosphorsäure, organische Sulfansäuren, z. B. p-Toluolsufonsäure, oder organische Carbonsäuren, z. B. Essigsäure und andere Niederalkansäuren. Basische Hydrolysemittel sind beispielsweise Metall- oder Ammoniumhydroxide, wie Alkalimetall- oder Erdalkalimetall- bzw. Ammoniumhydroxide, z. B. Natrium- Kalium-, Calcium- oder Ammoniumhydroxid, oder Alkalimetallcarbonate, z. B. Kalium- oder Natriumcarbonat, ferner organische Basen, wie tertiäre Amine, z. B. Triäthylamin. Lösungsmittel sind insbesondere mit Wasser mischbare Lösungsmittel, wie Niederalkanole, z. B. Methanol oder Äthanol, mit Wasser mischbare Äther oder

Ketone, z. B. Dioxan, Tetrahydrofuran oder Aceton, Dimethylformamid oder Dimethylsulfoxid.

In 4-Stellung niederalkyliertes Piperazinyl kann durch Behandlung mit einem N-Oxidationsmittel N-oxidiert werden.

Geeignete N-Oxidationsmittel sind beispielsweise Peroxyverbindungen, wie Wasserstoffperoxid, organische Hydroperoxide, z. B. tert.-Butylhydroperoxid, organische Persäuren, wie aromatische oder aliphatische Percarbonsäuren, z. B. m-Chlorperbenzoesäure, Peroxyessigsäure oder Monoperphthalsäure, oxidierende Schwermetallverbindungen, wie Chrom-VI- oder Mangan-IV- bzw. Mangan-VII-verbindungen, z. B. Chromtrioxid, Chromsäure, Mangandioxid oder Kaliumpermanganat, oxidierende anorganische Sauerstoffsäuren, wie Sauerstoffsäuren des Stickstoffs der Halogene oder Chalkogene, oder deren Anhydride oder Salze, z. B. Salpetersäure, Distickstofftetroxid, Selendioxid oder Natriummetaperjodat, ferner Ozon. Geeignete Lösungsmittel sind beispielsweise halogenierte Kohlenwasserstoffe, wie Halogenalkane, z. B. Kohlenstofftetrachlorid, Chloroform oder Methylenchlorid, oder Carbonsäuren, wie Alkansäuren, z. B. Essigsäure, oder deren Anhydride.

In einer bevorzugten Ausführungsform dieses Oxidationsverfahrens kann man beispielsweise Verbindungen der Formel I, worin $R_1$ für in 4-Stellung $C_1$-$C_4$-alkyliertes 1-Piperazinyl steht, durch Umsetzung mit einer organischen Persäure, z. B. mit m-Chlorperbenzoesäure, in einem Halogenalkan, z. B. in Chloroform, N-oxidieren.

Umgekehrt kann man in Verbindungen der Formel I, worin $R_1$ N-oxidiertes, durch $C_1$-$C_4$-Alkyl substituiertes 1-Piperazinyl ist, das N-oxidierte Ringstickstoffatome reduzieren. Die Reduktion erfolgt durch Behandeln mit üblichen Reduktionsmitteln, z. B. mit nascierendem oder katalytisch aktiviertem Wasserstoff, wie Eisen oder Zink und Säure, wie Salzsäure, oder mit Wasserstoff in Gegenwart von Raney-Nickel, vorteilhat in einem inerten Lösungsmittel, wie einem Niederalkanol, mit einer Phosphor-III-verbindung, wie einem Phosphin, z. B. Triphenylphosphin oder Tri-n-butylphosphin, oder einem Phosphorigsäureester, wie einem Triniederalkylphosphit, z. B. mit Trimethyl- oder Triäthylphosphit.

Erhaltene freie salzbildende Verbindungem der Formel I können in an sich bekannter Weise in Salze übergeführt werden. Säuren z. B. mit einer Base oder mit einem geeigneten Salz einer Carbonsäure und Basen mit einer Mineralsäure, üblicherweise in Gegenwart eines Lösungs- oder Verdünnungsmittel.

Erhaltene Salze können in an sich bekannter Weise in die freien Verbindungen umgewandelt werden, z. B. durch Behandeln mit einem sauren Reagens, wie einer Mineralsäure.

Die Verbindungen, inklusive ihre Salz, können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukt erhältlichen Verbindung ausgeht und die fehlenden Verfahrensschritte ausführt, oder einen Ausgangsstoff unter den Reaktionsbedingungen bildet oder in Form eines Derivates, gegebenenfalls eines Salzes, verwendet.

Beim Verfahren der vorliegenden Erfindung werden vorzugsweise solche Ausgangsstoffe verwendet, welche zu den eingangs als besonders wertvoll geschilderten Verbindungen führem. Neue Ausgangsstoffe und Verfahren zu ihrer Herstellung bilden ebenfalls einen Gegenstand der vorliegenden Erfindung.

Die neuen Verbindungen weisen wertvolle antiparasitäre Wirkungen, insbesondere gegen parasitäre Helminthen auf. Sie zeigen sie bei sehr guter Verträglichkeit; z. B. in Versuchstieren, wie Maus, Backenmaus (Saccostomus campestris), Ratte, Goldhamster, Mongolian Jird, (Meriones unguiculatus), Hund, Affe oder Huhn, Wirkungen gegen Trematoden, wie Fascioliden, z. B. Fasciola hepatica, und in erster Linie Schistosomen, z. B. Schistosoma mansoni, Schistosoma japonicum und Schistosoma haematobium, und gegen die Erreger der Filariose, z. B. Dipetalonema viteae und Litomosoides carinii. Beispielsweise weisen die Verbindungen der vorliegenden Erfindung bei der Behandlung von Goldhamstern mit einer 6 bis 8 Wochen alten Infektion von Schistosoma mansoni bei einmaliger Verabreichung p.o. (z. B. mit einer Magensonde) eine $ED_{50}$ ab etwa 30 - 200 mg/kg auf. Ebenso erweisen sich die neuen Verbindungen bei der Behandlung der Filariose im Mongolian Jird (Meriones unguiculatus) als macro- und microfilarizid und zwar bei fünfmaliger Verabreichung p.o. einer Dosis ab etwa 10-50 mg/kg sowohl bei Infektionen mit Dipetalonema viteae als auch mit Litomosoides carinii. Die neuen Verbindungen können dementsprechend zur Behandlung von Warmblütern bei Infektionen mit parasitären Helminthen, wie den obgenannten, insbesondere bei der Behandlung der Filariose verwendet werden.

Mit Hilfe der folgenden Versuchsdurchführung lässt sich beispielsweise die antischistosomale Aktivität von Verbindungen der Formel I am Beispiel der weissen Maus beziehungsweise des syrischen Hamsters ermitteln.

Erwachsene weisse Mäuse mit einem Gewicht von 20 bis 25 g werden durch subkutane Injektion von 80 Cercariae von Schistosoma mansoni pro Tier infestiert. Diese Cercariae werden sämtlich von derselben Gruppe von "positiven" Schecken (Species Biomphilaria glabrata) gewonnen und Gruppen von je 100 bis 150 Mäusen injiziert. Mit den syrischen Hamstern verfährt man in gleicher Weise.

Nachher wird das Auftreten von erwachsenenparasiten durch den "Miracidia-hatching-Test" positiv festgestellt. Die Versuchstiere werden willkürlich in Gruppen von jeweils 10 Individuen aufgeteilt, wobei 2 Gruppen als Kontrollgruppen dienen. Die verbleibenden Gruppen werden mit mindestens 3 verschieden abgestuften Einzeldosen der Testverbindungen behandelt. Gleichzeitig werden die Tiere einer Kontrollgruppe

getötet und autopsiert, um die durchschnittliche Wurmzahl bei Beginn der Beobachtungsphase zu bestimmen.

14 Tage nach der Behandlung wird wöchentlich die Zahl der ausgeschiedenen lebensfähigen Eier, die mit Hilfe des "Miracidia-hatching-Tests" ermittelt wurde, protokolliert. 6 Wochen nach der Behandlung werden alle Tiere einschliesslich der der unbehandelten Kontrollgruppe getötet und autopsiert. Die totale Zahl der gefundenen Schistosomen wird von jedem Tier ermittelt. Das Verhältnis der durchschnittlichen Wurmzahl jeder behandelten Gruppe zu dem Mittelwert der beiden Kontrollgruppen wird bestimmt und der $ED_{50}$-Wert (50 %ige Reduktion im Vergleich zu den Kontrollwerten) nach der Methode von L.C. Miller und M.L. Tainter (Proc. Soc. Exp. Biol. Med. 57, 261-264, 1944) ermittelt.

Zur Bestimmung der antifilarialen Wirkung von Verbindungen der Formel I wird folgendermassen vorgegangen wobei als Testtiere Meriones (M. unguiculatus) und vielzitzige Ratten (Mastomys natalensis) dienen:

Junge erwachsene Meriones und vielzitzige Ratten aus Zufallszuchten, die von 30 bis 50 g wiegen, werden durch Zeckenbisse der Species Bdellonyssus bacoti nach der Methode von F. Hawking und P. Swell (Brit. J. Pharmacol. 3, 285-296, 1948) infiziert. Die Infektionsbehälter werden in einem kontinuierlichen Fluss gehalten: Jeden Montag z. B. werden die Zecken mit Litomosoides carinii, welche sich im Larvenstadiun befinden, infiziert, wobei die Zecken 8 Stunden lang von dem peripheren Blut verschiedener Nager, welches zahlreiche Mikrofilarien enthält, sich ernähren können. Donnerstags werden nicht infizierte Nager 8 Stunden lang in den Behältern gehalten, wobei sie von frisch ausgeschlüpften, hauptsächlich aber von älteren Zecken gebissen werden, die metazyklische Larven ($L_3$) der parasitären Fadenwürmer enthalten. Die Populationsdichte wird auf einem Niveau von mindestens 50 und höchstens 200 Mikrofilarien gehalten. Nach 9-10 Wochen lassen sich erwachsene Parasiten durch Untersuchungen von 5 $mm^3$ Blutproben aus dem kapillaren Blutgefässystem, in welchem Mikrofilarien zirkulieren, nachweisen. Die Blutproben, denen Heparin zugesetzt wird, werden bei einer 100fachen Vergrösserung mikroskopisch untersucht und die Zahl der lebenden Mikrofilarien in 15 Feldern mit 1,8 mm Durchmesser bestimmt. Es werden lediglich solche Tiere für die weiteren Experimente ausgewählt, die mehr als 50 Mikrofilarien enthalten. Diese Tiere werden willkürlich in verschiedene Gruppen aufgeteilt und einzeln in Macrolon®-Käfigen gehalten. Gruppen mit jeweils 3 Tieren werden mit verschieden dosierten oralen Proben an 5 hintereinanderfolgenden Tagen mit den Testsubstanzen behandelt. Eine Gruppe dient als unbehandelte Kontrollgruppe.

Am selben Tag nach der Behandlung werden die Mikrofilarien in genau der gleichen Weise wie vor der Behandlung gezählt. Diese Zählungen werden periodisch 2, 4 und 6 Wochen nach der Behandlung durchgeführt, wobei die Gesamtzahl an lebenden und toten Mikrofilarien in jedem Tier ermittelt wird.

Die minimale effektive Dosis (MED) gegen Mikrofilarien ist diejenige Dosis, die zu einer mindestens 95 %igen Verminderung der Zahl von Mikrofilarien im zirkulierenden Blut 5 Tage nach der Behandlung führt. Diese Dosis wird üblicherweise in einer zweiten Versuchsserie verifiziert. Als minimale kurative Dosis (MCD) gegen Mikrofilarien wird die Dosis angenommen, die alle Mikrofilarien in allen behandelten Tieren während der sechswöchigen Beobachtung - nach der Behandlung - vernichtet (H.P.Striebel, An. N.Y. Acad. Sci. 160, 491-498, 1978). Für einige langsamwirkende Substanzen wird die Periode auf 8 Wochen ausgedehnt. Parallel zu der Wiederholung der Experimente zur Bestimmung der MED wird ebenfalls in einer zweiten Serie die MCD ermittelt.

Als weiterer Testparasit dient u.a. Dipetalonema viteae, der in denselben Nagerarten gehalten wird wie Litomosoides carinii. Weichzecken (Ornithodorus moubata) dienen als Zwischenwirte und werden nach der infizierenden Blutnahrung bei einer Temperatur von 28°C und einer relativen Feuchtigkeit von 70-80 % bis etwa 7 Wochen gehalten. Infizierte Larven werden gesammelt, indem die Zecken in einige Tropfen Hankscher Lösung auf Petrischalen getaucht werden. Die Zecken werden im Baerman-Apparat von den infizierten Larven im selben Medium getrennt.

M. natalensis und M. unguiculatus werden anschliessend mit 70 Larven pro Tier durch subkutane Injektion in den Nacken infiziert.

11 bis 13 Wochen nach der Infizierung werden die Tiere wie bei L. carinii auf Mikrofilariose untersucht. Lediglich Tiere, die mindestens 50 Mikrofilarien pro 10 $mm^3$ Blut aufweisen, werden für die Eperimente verwendet. Um das Risiko eines "therapeutischen Schocks" möglichst gering zu halten, werden keine Tiere verwendet, die länger als 13 Wochen infiziert sind.

Nach Auswertung dieser und weiterer Tests erweisen sich Verbindungen der Formel I als ausgezeichnete Mikro- und Macrofilaricide, z. B. bei lymphatischen Filariosen und bei der Onchocerose, sowie als Schistosomicide, wobei ein wirksamer Dosisbereich von täglich etwa 3 bis 80 mg/kg bei oraler Applikation über 5 Tage anzusetzen ist.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung zur Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikatonsweise ab.

Im Normalfall ist für einen etwa 75 kg schweren Warmblüter bei oraler Applikation eine ungefähre Tagesdosis von etwa 0,25-2,5 g, vorteilhaft in mehreren gleichen Teildosen verteilt, zu veranschlagen.

Die neuen pharmazeutischen Präparate enthalten z. B. von etwa 10 % bis etwa 80 %, vorzugsweise von etwa 20 % bis etwa 60 % des Wirkstoffs. Erfindungsgemässe pharmazeutische Präparate zur enteralen bzw.

parenteralen Verabreichung sind z. B. solche in Dosiseinheitsformen wie Dragées, Tabletten, Kapseln oder Suppositorien, ferner Ampullen. Diese werden in an sich bekannter Weise, z. B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Starinsäure oder Salze davon, wie Magnesium- oder Calciumstearat, und/oder Polyäthylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaftresistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichnacher, wie Glycerin oder Sorbitol. Die Steckkapseln können den Wirkstoff in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugseise in geeigneten Flüssigkeiten, wie fetten Ölen, Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten; als Grundmassenstoffe kommen z. B. flüssige Triglyceride, Polyäthylenglykole oder Paraffinkohlenwasserstoff in Frage.

Zur parenteralen Verabreichung eigenen sich in erster Linie wässrige Lösungen eines Wirkstoffs in wasserlöslicher Form, z. B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z. B. Sesamöl, oder synthetische Fettsäureester, z. B. Äthyloleat oder Triglyceride, verwendet, oder wässrige Injektionssuspensionen, welche viskositätserhöhende Stoffe, z. B. Natrium-carboxymethylcellulose, Sorbit und/oder Dextran und gegebenenfalls auch Stabilisatoren enthalten.

Tinkturen und Lösungen weisen meistens eine wässerigäthanolische Grundlage auf, der u.a. Polyalkohole, z. B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglycolen, d.h. im wässrigen Gemisch lösliche, lipophile Substanzen als Ersatz für die durch den Alkohol entzogenen Fettsubstanzen, und, falls notwendig, andere Hilfs- und Zusatzmittel beigegeben sind.

Die vorliegende Erfindung betrifft ebenfalls die Verwendung der Verbindungen der Formeln I und II und der Salze von solchen Verbindungen mit salzbildenden Eigenschaften, vorzugsweise zur Bekämpfung parasitierender Helminthen, in erster Linie solcher der vorstehend genannten Familien.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: 70 g (0,28 Mol) 2-tert.-Butyl-6-isothiocyanao-benzthiazol werden in 1200 ml Äther gelöst und tropfenweise unter Rühren mit 33 g (0,33 Mol) 4-Methylpiperazin versetzt. Das Gemisch wird 1 Stunde gerührt, abfiltriert und zuerst mit Äther, dann mit Petroläther gewaschen. Man erhält das 2-tert.-Butyl-6-[(4-methylpiperazin-1-yl)-thiocarbonylamino]-benzthiazol vom Smp. 176-177°:

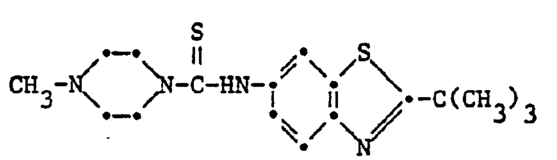 .

In analoger Weise kann man auch 2-tert.-Butyl-5-methoxy-6-[(4-methylpiperazin-1-yl)-thiocarbonyl-amino]-benzthiazol, Smp. 150-151°,

2-tert.-Butyl-5-chlor-6-[(4-methylpiperazin-1-yl)-thiocarbonyl-amino]-benzthiazol, Smp. 158-160°, und

2-tert.-Butyl-5-methyl-6-[(4-methylpiperazin-1-yl)-thiocarbonyl-amino]-benzthiazol, Smp. 185-187° herstellen.

Beispiel 2: 4,33 g (0,183 Mol) Natrium werden unter Stickstoff in 300 ml Äthanol gelöst. Dann werden 17,6 g (0,094 Mol) N-Methylpiperazin-N-oxid-dihydrochlorid hinzugegeben. Nach 15 Minuten wird das gebildete Natriumchlorid abfiltriert und mit Äthanol gewaschen. Das Filtrat wird in eine Suspension von 24 g (0,085 Mol) 2-tert.-Butyl-6-isothiocyano-5-methyl-benzthiazol in 120 ml Äthanol bei Raumtemperatur eingetropft. Dann wird 2 Stunden gerührt und das Äthanol unter Vakuum abdestilliert. Der Rückstand wird mit Wasser und Methylenchlorid extrahiert. Die Methylenchloridbase (100 ml) wird abgetrennt, mit Natriumsulfat getrocknet, filtriert, mit 100 ml Petroläther verdünnt und auf 0° gekühlt: Nach Filtration erhält man das 2-tert.-Butyl-5-methyl-6-[(4-methylpiperazin-4-oxid-1-yl)-thiocarbonylamino]-benzthiazol vom Smp. 129-131°.

In analoger Weise kann man auch 2-tert.-Butyl-5-methoxy-6-[(4-methylpiperazin-4-oxid-1-yl)-thiocarbonylamino]-benzthiazol, Smp. 144°,

2-tert.-Butyl-5-chlor-6-[(4-methylpiperazin-4-oxid-1-yl)-thiocarbonylamino]-benzthiazol, Smp. 119-121° herstellen.

Beispiel 3: 21 g (0,076 Mol) 2-tert.-Butyl-6-isothiocyano-5-methoxy-benzthiazol werden in 400 ml Aceton gelöst und tropfenweise mit einer Lösung von 12 g (0,092 Mol) N-(2-Hydroxyäthyl)-piperazin in 40 ml Aceton versetzt. Das Gemisch wird 2 Stunden gerührt, auf 0° gekühlt, abfiltriert und mit Aceton, dann mit Äther und zuletzt mit Petroläther gewaschen. Man erhält das 2-tert.-Butyl-5-methoxy-6-{[4-(2-hydroxyäthyl)-piperazin-1-yl]-thiocarbonylamino]}-benzthiazol vom Smp. 161-162°.

In analoger Weise kann man auch 2-tert.-Butyl-6-{[4-(2-hydroxyäthyl)-piperazin-1-yl]-thiocarbonylamino}-benzthiazol, Smp. 172-175°, und 2-iso-Propyl-6-{[4-(2-hydroxyäthyl)-piperazin-1-yl]-thiocarbonylamino}-benzthiazol, Smp. 172-175° herstellen.

Beispiel 4: 27,8 g (0,10 Mol) 2-tert.-Butyl-6-isothiocyano-5-methoxy-benzthiazol werden in 150 ml Dimethylformamid gelöst und mit 14 g (0,13 Mol) 3-Mercaptopropionsäure versetzt. Die Lösung wird unter Stickstoff 2 Stunden bei Raumtemperatur gerührt, dann auf 2 kg Eis gegossen und gerührt, bis das Produkt fest geworden ist. Es wird abfiltriert, mit Wasser gewaschen und in 700 ml Toluol erwärmt, von Wasser abdekantiert, über Natriumsulfat getrocknet, mit Aktivkohle geklärt und filtriert. Das Filtrat wird bei 30-40° mit ca. 700 ml Petroläther verdünnt und langsam auf 5° gekühlt, abfiltriert und mit Petroläther nachgewaschen. Man erhält N-[2-tert.-Butyl-5-methoxy-benzthiazol-6-yl]-dithiocarbaminsäure-S-(2-carboxyäthyl)-ester vom Smp. 154-156°:

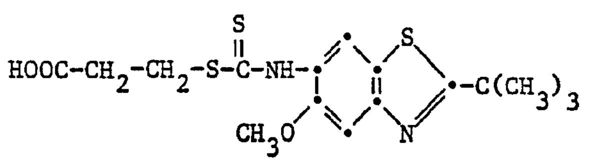
.

In analoger Weise kann man auch N-[2-tert.-Butyl-benzthiazol-6-yl]-dithiocarbaminsäure-S-(2-carboxyäthyl)-ester, Smp. 135-137°,

N-[2-tert.-Butyl-5-chlor-benzthiazol-6-yl]-dithiocarbaminsäure-S-(2-carboxyäthyl)-ester, Smp. 95-97°, und

N-[2-tert.-Butyl-5-methyl-benzthiazol-6-yl]-dithiocarbaminsäure-S.-(2-carboxyäthyl)-ester, Smp. 155-158° herstellen.

Beispiel 5: In analoger Weise wie in den Beispielen 1-4 beschrieben kann man ferner herstellen:

N-[2-tert.-Butyl-5-methoxy-benzthiazol-6-yl]-dithiocarbaminsäure-S-(2-carboxypropyl)-ester, Fp. 136-140°,

N-[2-tert.-Butyl-5-methoxy-benzthiazol-6-yl]-dithiocarbaminsäure-S-carboxymethyl-ester, Fp. 231-233°,

N-[2-tert.-Butyl-5-methoxy-benzthiazol-6-yl]-thiocarbaminsäure-O-(2-carboxyäthyl)-ester, Fp. 173 - 176°.

Beispiel 6: In analoger Weise wie in den Beispielen 1-4 oder nach einem der in der Beschreibung beschriebenen Herstellungsverfahren für Verbindungen der Formel I kann man ferner herstellen:

N-[2-tert.-Butyl-5-methoxy-benzthiazol-6-yl]-thiocarbaminsäure-S-(2-carboxyäthyl)-ester, Fp. 231 - 233°,

2-tert.-Butyl-5-methoxy-6-[(4-methylpiperazin-1-yl)-carbonylamino]-benzthiazol, Fp. 176 - 179°,

2-tert.-Butyl-5-methyl-6-[(4-methylpiperazin-1-yl)-thiocarbonylamino]-benzoxazol, Fp. 195 - 197°,

2-tert.-Butyl-5-chlor-6-[(4-methylpiperazin-1-yl)-thiocarbonylamino]-benzoxazol, Fp. 181 - 182°,

2-tert.-Butyl-5-methoxy-6-[(4-methylpiperazin-1-yl)-thiocarbonylamino]-benzoxazol,

2-tert.-Butyl-5-methylthio-6-[(4-methylpiperazin-1-yl)-thiocarbonylamino]-benzoxazol,

2-tert.-Butyl-6-[(4-methylpiperazin-1-yl)-thiocarbonylamino]-benzoxazol,

2-tert.-Butyl-5-methyl-6-[(4-methylpiperazin-4-oxid-1-yl)-thiocarbonylamino]-benzoxazol, Fp. 141 - 143°,

2-tert.-Butyl-5-chlor-6-[(4-methylpiperazin-4-oxid-1-yl)-thiocarbonylamino]-benzoxazol, Fp. 135 - 137°,

2-tert.-Butyl-5-methoxy-6-[(4-methylpiperazin-4-oxid-1-yl)-thiocarbonylamino]-benzoxazol,

2-tert.-Butyl-5-methylthio-6-[(4-methylpiperazin-4-oxid-1-yl)-thiocarbonylamino]-benzoxazol,

2-tert.-Butyl-6-[(4-methylpiperazin-4-oxid-1-yl)-thiocarbonylamino]-benzoxazol,

N-[2-tert.-Butyl-5-methyl-benzoxazol-6-yl]-dithiocarbaminsäure-S-(2-carboxyäthyl)-ester, Fp. 160 - 163°,

N-[2-tert.-Butyl-5-chlor-benzoxazol-6-yl]-dithiocarbaminsäure-S-(2-carboxyäthyl)-ester, Fp. 85 - 88°,

N-[2-tert.-Butyl-5-methoxy-benzoxazol-6-yl]-dithiocarbaminsäure-S-(2-carboxyäthyl)-ester,

N-[2-tert.-Butyl-5-methylthio-benzoxazol-6-yl]-dithiocarbaminsäure-S-(2-carboxyäthyl)-ester,

2-tert.-Butyl-5-[(4-methylpiperazin-1-yl)-thiocarbonylamino]-benzoxazol

N-[2-tert.-Butyl-benzoxazol-5-yl]-dithiocarbaminsäure-S-(2-carboxyäthyl)-ester,

Beispiel 7: Tabletten, enthaltend 2-tert.-Butyl-6-[(4-methylpiperazin-1-yl)-thiocarbonylamino]-benzthiazol, können wie folgt hergestellt werden:

| Zusammensetzung (für 10000 Tabletten) | |
|---|---|
| 2-tert.-Butyl-6-[(4-methylpiperazin-1-yl)-thiocarbonylamino]-benzthiazol | 5000 g |
| Weizenstärke | 790 g |
| Stearinsäure | 30 g |
| Magnesiumstearat | 30 g |
| Talk | 400 g |
| Wasser | q.s. |

Ein Gemisch des 2-tert.-Butyl-6-[(4-methylpiperazin-1-yl)-thiocarbonylamino]-benzthiazol und 500 g Weizenstärke wird mit etwa 1300 g entmineralisiertem Wasser verkleistert und weiteren 600 g entmineralisiertem Wasser gleichmässig befeuchtet. Das Gemisch wird zu einer schwach plastischen Masse geknetet und durch ein Sieb von etwa 3 mm Maschenweite getrieben. Das Granulat wird anschliessend getrocknet und erneut durch ein Sieb geschlagen. Dem trockenen und auf eine einheitliche Korngrösse gebrachten Granulat werden das Magnesiumstearat, die Stearinsäure, der Talk und 290 g Weizenstärke zugemischt und das Gemisch zu Tabletten von 0,625 g verpresst.

In analoger Weise kann man auch Tabletten, enthaltend eine andere Verbindung gemäss Beispiel 1 oder eine Verbindung gemäss Beispiele 2 bis 5 als Wirkstoff, herstellen.

Beispiel 8: In analoger Weise wie in Beispiel 7 beschrieben kann man auch Tabletten, enthaltend eine der in Beispiel 6 genannten Verbindungen, herstellen.

**Patentansprüche**

1. Eine Verbindung der Formel

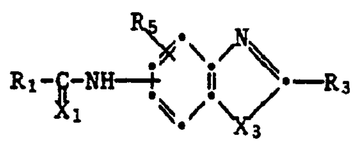

(I),

worin $R_1$ 4-$C_1$-$C_4$-Alkylpiperazin-1-yl, 4-$C_1$-$C_4$-Alkylpiperazin-4-oxid-1-yl, 4-(Hydroxy-$C_2$-$C_4$-alkyl)-piperazin-1-yl, worin die Hydroxygruppe in höherer als der α-Stellung gebunden ist, oder eine gegebenenfalls mit einem $C_1$-$C_4$-Alkanol veresterte Carboxy-$C_1$-$C_4$-alkoxy- oder Carboxy-$C_1$-$C_4$-alkylthio-Gruppe, $X_1$ Sauerstoff oder Schwefel, $R_5$ Wasserstoff, $C_1$-$C_4$-Alkyl, $C_1$-$C_4$-Alkoxy, $C_1$-$C_4$-Alkylthio oder Halogen, $R_3$ α-verzweigtes $C_3$-$C_4$-Alkyl oder $C_3$-$C_8$-Cycloalkyl und $X_3$ Sauerstoff oder Schwefel bedeuten, und Salze davon.

2. Eine Verbindung gemäss Anspruch 1 der Formel I, worin $R_5$ Wasserstoff, $C_1$-$C_4$-Alkyl, $C_1$-$C_4$-Alkoxy oder Halogen bedeutet, und Salze davon.

3. Eine Verbindung gemäss Anspruch 1 oder 2, worin die Gruppe $R_1$-C(=$X_1$)-NH- in 6-Stellung steht.

4. Eine Verbindung gemäss Anspruch 1 oder 2, worin $R_5$ Wasserstoff bedeutet oder in 5-Stellung steht und Methyl oder Methoxy ist.

5. Eine Verbindung gemäss Anspruch 1 oder 2, worin $X_3$ Sauerstoff ist.

6. Eine Verbindung gemäss Anspruch 1 oder 2, worin $X_3$ Schwefel ist.

7. Eine Verbindung gemäss Anspruch 1 oder 2, worin $R_3$ Isopropyl ist.

8. Eine Verbindung gemäss Anspruch 1 oder 2, worin $R_3$ tert-Butyl ist.

9. 2-tert-Butyl-6-[(4-methylpiperazin-1-yl)-thiocarbonylamino]-benzthiazol oder ein Salz davon.

10. 2-tert-Butyl-5-methoxy-6-[(4-methylpiperazin-1-yl)-thiocarbonylamino]-benzthiazol oder ein Salz davon.

11. 2-tert-Butyl-5-chlor-6-[(4-methylpiperazin-1-yl)-thiocarbonylamino]-benzthiazol oder ein Salz davon.

12. 2-tert-Butyl-5-methyl-6-[(4-methylpiperazin-1-yl)-thiocarbonylaminol]-benzthiazol oder ein Salz davon.

13. 2-tert-Butyl-6-[(4-methylpiperazin-4-oxid-1-yl)-thiocarbonylamino]-benzthiazol oder ein Salz davon.

14. 2-tert-Butyl-5-methoxy-6-[(4-methylpiperazin-4-oxid-1-yl)-thiocarbonylamino]-benzthiazol oder ein Salz davon.

15. 2-tert-Butyl-5-chlor-6-[(4-methylpiperazin-4-oxid-1-yl)-thiocarbonylamino]-benzthiazol oder ein Salz davon.

16. 2-tert-Butyl-5-methyl-6-[(4-methylpiperazin-4-oxid-1-yl)-thiocarbonylamino]-benzthiazol oder ein Salz davon.

17. 2-tert-Butyl-5-methoxy-6-([4-(2-hydroxyäthyl)-piperazin-1-yl]-thiocarbonylamino)-benzthiazol oder ein Salz davon.

18. 2-tert-Butyl-6-{[4-(2-hydroxyäthyl)-piperazin-1-yl]-thiocarbonylamino}-benzthiazol oder ein Salz davon.

19. 2-Isopropyl-6-{[4-(2-hydroxyäthyl)-piperazin-1-yl]-thio-carbonylamino}-benzthiazol oder ein Salz davon.

20. N-[2-tert-Butyl-5-methoxy-benzthiazol-6-yl]-dithiocarbaminsäure-S-(2-carboxyäthyl)-ester oder ein Salz davon.

21. N-[2-tert-Butyl-benzthiazol-6-yl]-dithiocarbaminsüure-S-(2-carboxyäthyl)-ester oder ein Salz davon.

22. N-[2-tert-Butyl-5-chlor-benzthiazol-6-yl]-dithiocarbaminsäure-S-(2-carboxyäthyl)-ester oder ein Salz davon.

23. N-[2-tert-Butyl-5-methyl-benzthiazol-6-yl]-dithiocarbaminsäure-S-(2-carboxyäthyl)-ester oder ein Salz davon.

24. N-[2-tert-Butyl-5-methoxy-benzthiazol-6-yl]-dithiocarbaminsäure-S-(2-carboxypropyl)-ester oder ein Salz davon.

25. N-[2-tert-Butyl-5-methoxy-benzthiazol-6-yl]-dithiocarbaminsäure-S-carboxymethyl-ester oder ein Salz davon.

26. N-[2-tert-Butyl-5-methoxy-benzthiazol-6-yl]-thiocarbaminsäure-O-(2-carboxyäthyl)-ester.

27. N-[2-tert-Butyl-5-methoxy-benzthiazol-6-yl]-thiocarbaminsäure-S-(2-carboxyäthyl)-ester oder ein Salz davon.

28. 2-tert-Butyl-5-methoxy-6-[(4-methylpiperazin-1-yl)-carbonylamino]-benzthiazol oder ein Salz davon.

29. 2-tert-Butyl-5-methyl-6-[(4-methylpiperazin-1-yl)-thiocarbonylamino]-benzoxazol oder ein Salz davon.

30. 2-tert-Butyl-5-chlor-6-[(4-methylpiperazin-1-yl)-thiocarbonylamino]-benzoxazol oder ein Salz davon.

31. 2-tert-Butyl-5-methyl-6-[(4-methylpiperazin-4-oxid-1-yl)-thiocarbonylamino]-benzoxazol oder ein Salz davon.

32. 2-tert-Butyl-5-chlor-6-[(4-methylpiperazin-4-oxid-1-yl)-thiocarbonylamino]-benzoxazol oder ein Salz davon.

33. N-[2-tert-Butyl-5-methyl-benzoxazol-6-yl]-dithiocarbaminsäure-S-(2-carboxyäthyl)-ester oder ein Salz davon.

34. N-[2-tert-Butyl-5-chlor-benzoxazol-6-yl]-dithiocarbaminsäure-S-(2-carboxyäthyl)-ester oder ein Salz davon.

35. Verfahren zur Herstellung von Verbindungen der Formel I gemäss Anspruch 1 und ihrer Salze, dadurch gekennzeichnet, dass man

a) Verbindungen der Formel II und III

$R_1—Y_1$ (II) und 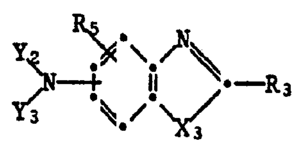 (III) ,

worin $Y_1$ Wasserstoff bedeutet und $Y_2$ und $Y_3$ gemeinsam die Gruppe der Formel $=C=X_1$ bedeuten, oder einer der Reste $Y_1$ und $Y_2$ die Gruppe der Formel $-C(=X_1)-Y_4$ bedeutet, wobei $Y_4$ für einen abspaltbaren Rest steht, und der andere Rest und $Y_3$ Wasserstoff darstellen miteinander kondensiert, oder

b) in einer Verbindung der Formel IV

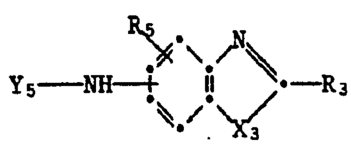 (IV),

worin $Y_5$ einen in die Gruppe der Formel $R_1-C(=X_1)-$ überführbaren Rest bedeutet, oder in einem Salz davon, $Y_5$ in die Gruppe der Formel $R_1-C(=X_1)-$ überführt, oder

c) eine Verbindung der Formel

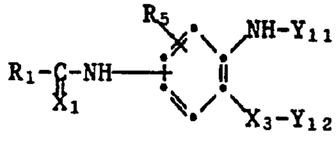 (V),

worin einer der Reste $Y_{11}$ und $Y_{12}$ die Gruppe der Formel $-C(=Y_{13})-R_3$ und der andere einen abspaltbaren Rest bedeutet und $Y_{13}$ für gegebenenfalls funktionell abgewandeltes Oxo steht, oder ein Salz davon cyclisiert und gewünschtenfalls eine erfindungsgemäss erhältliche Verbindung in eine andere Verbindung der Formel I umwandelt und/oder ein erhältliches Salz in die freie Verbindung oder in ein anderes Salz überführt.

36. Die nach dem Verfahren des Anspruchs 35 erhältlichen Verbindungen.

37. Eine Verbindung gemäss Anspruch 1 zur Anwendung in einem Verfahren zur therapeutischen Behandlung des menschlichen oder tierischen Körpers.

38. Ein pharmazeutisches Präparat enthaltend eine Verbindung gemäss Anspruch 1 neben üblichen pharmazeutischen Hilfs- und Trägerstoffen.

## Claims

1. A compound of formula

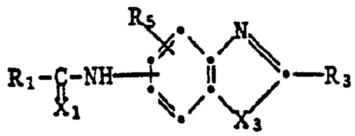

(I),

wherein $R_1$ is 4-alkylpiperazin-1-yl containing 1 to 4 carbon atoms in the alkyl moiety, 4-alkylpiperazin-4-oxid-1-yl containing 1 to 4 carbon atoms in the alkyl moiety, 4-(hydroxyalkyl)piperazin-1-yl containing 2 to 4 carbon atoms in the alkyl moiety, and wherein the hydroxy group is in a position higher than the α-position, or is a carboxyalkoxythio or carboxyalkylthio group, each containing 1 to 4 carbon atoms in the alkoxy or alkyl moiety respectively, which group may be esterified with a $C_1$-$C_4$alkanol, $X_1$ is oxygen or sulfur, $R_5$ is hydrogen, $C_1$-$C_4$alkyl, $C_1$-$C_4$alkoxy, $C_1$-$C_4$alkylthio or halogen, $R_3$ is an α-branched $C_3$-$C_4$alkyl or $C_3$-$C_8$cycloalkyl group, and $X_3$ is oxygen or sulfur, or a salt therof:

2. A compound according to claim 1 of formula I, wherein $R_5$ is hydrogen, $C_1$-$C_4$alkyl, $C_1$-$C_4$alkoxy or halogen, or a salt thereof.

3. A compound according to either claim 1 or claim 2, wherein the $R_1$-$C(=X_1)$-NH- group is in 6-position.

4. A compound according to either claim 1 or claim 2, wherein $R_5$ is hydrogen or is in 5-position, and is methyl or methoxy.

5. A compound according to either claim 1 or claim 2, wherein $X_3$ is oxygen.

6. A compound according to either claim 1 or claim 2, wherein $X_3$ is sulfur.

7. A compound according to either claim 1 or claim 2, wherein $R_3$ is isopropyl.

8. A compound according to either claim 1 or claim 2, wherein $R_3$ is tert-butyl.

9. 2-tert-Butyl-6-[(4-methylpiperazin-1-yl)thiocarbonylamino]benzthiazole or a salt thereof.

10. 2-tert-Butyl-5-methoxy-6-[(4-methylpiperazin-1-yl)thiocarbonylamino]benzthiazole or a salt thereof.

11. 2-tert-Butyl-5-chloro-6-[(4-methylpiperazin-1-yl)-thiocarbonylamino]benzthiazole or a salt thereof.

12. 2-tert-Butyl-5-methyl-6-[(4-methylpiperazin-1-yl)-thiocarbonylamino]benzthiazole or a salt thereof.

13. 2-tert-Butyl-6-[(4-methyl-4-oxido-piperazin-1-yl)-thiocarbonylamino]benzthiazole or a salt thereof.

14. 2-tert-Butyl-5-methoxy-6-[(4-methyl-4-oxido-piperazin-1-yl)thiocarbonylacylamino]benzthiazole or a salt thereof.

15. 2-tert-Butyl-5-chloro-6-[(4-methyl-4-oxido-piperazin-1-yl)thiocarbonylamino]benzthiazole or a salt thereof.

16. 2-tert-Butyl-5-methyl-6-[(4-methyl-4-oxido-piperazin-1-yl)thiocarbonylamino]benzthiazole or a salt thereof.

17. 2-tert-Butyl-5-methoxy-6-{[4-(2-hydroxyethyl)-piperazin-1-yl]thiocarbonylamino}benzthiazole or a salt thereof.

18. 2-tert-Butyl-6-{[4-(2-hydroxyethyl)-piperazin-1-yl)-thiocarbonylamino}benzthiazole or a salt thereof.

19. 2-Isopropyl-6-{[4-(2-hydroxyethyl)-piperazin-1-yl]-thiocarbonylamino}benzthiazole or a salt thereof.

20. S-(2-Carboxyethyl) N-[2-tert-butyl-5-methoxy-benzthiazol-6-yl]-dithiocarbamate or a salt thereof.

21. S-(2-Carboxyethyl) N-[2-tert-butylbenzthiazol-6-yl]-dithiocarbamate or a salt thereof.

22. S-(2-Carboxyethyl) N-[2-tert-butyl-5-chlorobenzthiazol-6-yl]-dithiocarbamate or a salt thereof.

23. S-(2-Carboxyethyl) N-[2-tert-butyl-5-methylbenzthiazol-6-yl] dithiocarbamate or a salt thereof.

24. S-(2-Carboxypropyl) N-[2-tert-butyl-5-methoxy-benzthiazol-6-yl]-dithiocarbamate or a salt thereof.

25. S-Carboxymethyl N-[2-tert-butyl-5-methoxybenzthiazol-6-yl]-dithiocarbamate or a salt thereof.

26. O-(2-Carboxyethyl) N-[2-tert-butyl-5-methoxybenzthiazol-6-yl]thiocarbamate or a salt thereof.

27. S-(2-Carboxyethyl) N-[2-tert-butyl-5-methoxybenzthiazol-6-yl]thiocarbamate or a salt thereof.

28. 2-tert-Butyl-5-methoxy-6-[(4-methylpiperazin-1-yl)-carbonylamino]benzthiazole or a salt thereof.

29. 2-tert-Butyl-5-methyl-6-[(4-methylpiperazin-1-yl)-thiocarbonylamino]benzoxazole or a salt thereof.

30. 2-tert-Butyl-5-chloro-6-[(4-methylpiperazin-1-yl)-thiocarbonylamino]benzoxazole or a salt thereof.

31. 2-tert-Butyl-5-methyl-6-[(4-methylpiperazin-4-oxid-1-yl)thiocarbonylamino]benzoxazole or a salt thereof.

32. 2-tert-Butyl-5-chloro-6-[(4-methylpiperazin-4-oxid-1-yl)thiocarbonylamino]benzoxazole or a salt thereof.

33. S-(2-Carboxyethyl) N-[2-tert-butyl-5-methylbenzoxazol-6-yl)-dithiocarbamate or a salt thereof.

34. S-(2-Carboxyethyl) N-[2-tert-butyl-5-chlorobenzoxazol-6-yl]-dithiocarbamate or a salt thereof.

35. A process for the preparation of a compound of formula I according to claim 1, or a salt thereof, which

comprises

a) condensing compounds of formulae II and III $R_1-Y_1$ (II) and (III),

$R_1$—$Y_1$ (II) and 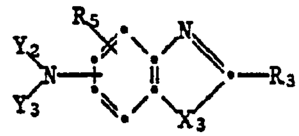 (III) ,

wherein $Y_1$ is hydrogen and $Y_2$ and $Y_3$ together are a group of formula $=C=X_1$ or one of $Y_1$ and $Y_2$ is a group of formula $-C(=X_1)-Y_4$, in which $Y_4$ is a removable radical and the other and $Y_3$ are hydrogen, or salts thereof, or

b) in a compound of formula IV

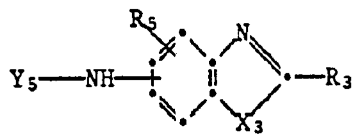 (IV),

wherein $Y_5$ is a radical that can be converted into a group of formula $R_1-C(=X_1)-$, or in a salt thereof, $Y_5$ is converted into a group of the formula $R_1-C(=X_1)-$, or

c) cyclising a compound of formula V

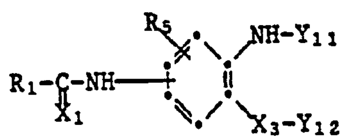 (V),

wherein one of $Y_{11}$ and $Y_{12}$ is a group of formula $-C(=Y_{13})-R_3$ and the other is a removable radical, and $Y_{13}$ is free or functionally modified oxo, or a salt thereof, and, if desired, converting a resultant compound into another compound of formula I and/or a resultant salt into the free compound or into another salt.

36. A compound obtainable by the process as claimed in claim 35.

37. A compound according to claim 1 for use in a method for the therapeutic treatment of the human or animal body.

38. A pharmaceutical composition containing a compound as claimed in claim 1, together with conventional pharmaceutical excipients and carriers.

**Revendications**

1. Un composé de formule

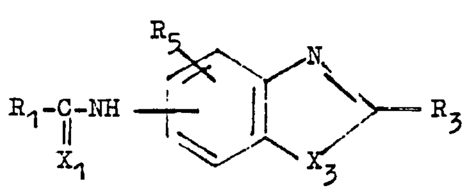 (I),

dans laquelle $R_1$ représente un groupe 4-(alkyle en C 1-C 4)-pipérazine-1-yle, 4-(alkyle en C 1-C 4)-pipérazine--4-oxyde-1-yle, 4-(hydroxyalkyle en C 2-C 4)-pipérazine-1-yle dans lequel le groupe hydroxy est dans une position supérieure à la position α, ou un groupe carboxyalcoxy en C 1-C 4 ou carboxy-alkylthio en C 1-C 4 éventuellement estérifié par un alcanol en C 1-C 4, $X_1$ représente l'oxygène ou le soufre, $R_5$ représente l'hydrogène, un groupe alkyle en C 1-C 4, alcoxy en C 1-C 4, alkylthio en C 1-C 4 ou un halogène, $R_3$ représente un groupe alkyle en C 3-C 4 ramifié en α ou cycloalkyle en C 3-C 8 et $X_3$ représente l'oxygène ou le soufre, et ses sels.

2. Un composé selon la revendication 1, de formule I dans laquelle $R_5$ représente l'hydrogène, un groupe alkyle en C 1-C 4, alcoxy en C 1-C 4 ou un halogène, et ses sels.

3. Un composé selon la revendication 1 ou 2, dans lequel le groupe $R_1$-C(=$X_1$)-NH- est en position 6.

4. Un composé selon la revendication 1 ou 2 dans lequel $R_5$ représente l'hydrogène ou bien un groupe méthyle ou méthoxy en position 5.

5. Un composé selon la revendication 1 ou 2, dans lequel $X_3$ représente l'oxygène,

6. Un composé selon la revendication 1 ou 2, dans lequel $X_3$ représente le soufre.

7. Un composé selon la revendication 1 ou 2,, dans lequel $R_3$ représente un groupe isopropyle.

8. Un composé selon la revendication 1 ou 2, dans lequel $R_3$ représente un groupe tert.-butyle.

9. Le 2-tert.-butyl-6-[(4-méthylpipérazine-1-yl)-thiocarbonylamino]-benzothiazole ou l'un de ses ssels.

10. Le 2-tert.-butyl-5-méthoxy-6-[(4-méthylpipérazine-1-yl)-thiocarbonylamino]-benzothiazole ou l'un de ses sels.

11. Le 2-tert.-butyl-5-chloro-6-[(4-méthylpipérazine-1-yl)-thiocarbonylamino]-benzothiazole ou l'un de ses sels.

12. Le 2-tert.-butyl-5-méthyl-6-[(4-méthylpipérazine-1-yl)-thiocarbonylamino]-benzothiazole ou l'un de ses sels.

13. Le 2-tert.-butyl-6-[(4-méthylpipérazine-4-oxyde-1-yl)-thiocarbonylamino]-benzothiazole ou l'un de ses sels.

14. Le 2-tert.-butyl-5-méthoxy-6-[(4-méthylpipérazine-4-oxyde-1-yl)-thiocarbonylamino]-benzothiazole ou l'un de ses sels.

15. Le 2-tert.-butyl-5-chloro-6-[(4-méthylpipérazine-4-oxyde-1-yl)-thiocarbonylamino]-benzothiazole ou l'un de ses sels.

16. Le 2-tert.-butyl-5-méthyl-6-[(4-méthylpipérazine-4-oxyde-1-yl)-thiocarbonylamino]-benzothiazole ou l'un de ses sels.

17. Le 2-tert.-butyl-5-méthoxy-6-{[4-(2-hydroxyéthyl)-pipérazine-1-yl]-thiocarbonylamino}-benzothiazole ou l'un de ses sels.

18. Le 2-tert.-butyl-6-{[4-(2-hydroxyéthyl)-pipérazine-1-yl]-thiocarbonylamino}-benzothiazole ou l'un de ses sels.

19. Le 2-isopropyl-6-{[4-(2-hydroxyéthyl)-pipérazine-1-yl]-thiocarbonylamino}-benzothiazole ou l'un de ses sels.

20. Le N-[2-tert.-butyl-5-méthoxy-benzothiazole-6-yl]-dithiocarbamate de S-(2-carboxyéthyle) ou l'un de ses sels.

21. Le N-[2-tert.-butyl-benzothiazole-6-yl]-dithiocarbamate de S-(2-carboxyéthyle) ou l'un de ses sels.

22. Le N-[2-tert.-butyl-5-chloro-benzothiazole-6-yl]-dithiocarbamate de S-(2-carboxyéthyle) ou l'un de ses sels.

23. Le N-[2-tert.-butyl-5-méthyl-benzothiazole-6-yl]-dithiocarbamate de S-(2-carboxyéthyle) ou l'un de ses sels.

24. Le N-[2-tert.-butyl-5-méthoxy-benzothiazole-6-yl]-dithiocarbamate de S-(2-carboxypropyle) ou l'un de ses sels.

25. Le N-[2-tert.-butyl-5-méthoxy-benzothiazole-6-yl]-dithiocarbamate de S-carboxyméthyle ou l'un de ses sels.

26. Le N-[2-tert.-butyl-5-méthoxy-benzothiazole-6-yl]-thiocarbamate d'O-(2-carboxyéthyle).

27. Le N-[2-tert.-butyl-5-méthoxy-benzothiazole-6-yl]-thiocarbamate de S-(2-carboxyéthyle) ou l'un de ses sels.

28. Le 2-tert.-butyl-5-méthoxy-6-[(4-méthylpipérazine-1-yl)-carbonylamino]-benzothiazole ou l'un de ses sels.

29. Le 2-tert.-butyl-5-méthyl-6-[(4-méthylpipérazine-1-yl)-thiocarbonylanino]-benzoxazole ou l'un de ses sels.

30. Le 2-tert.-butyl-5-chloro-6-[(4-méthylpipérazine-1-yl)-thiocarbonylamino]-benzoxazole ou l'un de ses sels.

31. Le 2-tert.-butyl-5-méthyl-6-[(4-méthylpipérazine-4-oxyde-1-yl)-thiocarbonylamino]-benzoxazole ou l'un de ses sels.

32. Le 2-tert.-butyl-5-chloro-6-[(4-méthylpipérazine-4-oxyde-1-yl)-thiocarbonylamino]-benzoxazole ou l'un de ses sels.

33. Le N-[2-tert.-butyl-5-méthyl-benzoxazole-6-yl]-dithiocarbamate de S-(2-carboxyéthyle) ou l'un de ses sels.

34. Le N-[2-tert.-butyl-5-chloro-benzoxazole-6-yl]-dithiocarbamate de S-(2-carboxyéthyle) ou l'un de ses sels.

35. Procédé de préparation des composés de formule I selon la revendication 1 et de leurs sels, caractérisé en ce que:

a) on condense entre eux des composés de formules II et III

$R_1—Y_1$ (II)

$R_1 —— Y_1$ (II)

et

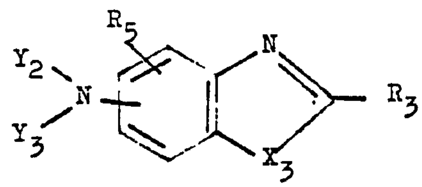

(III),

dans lesquelles $Y_1$ représente l'hydrogène et $Y_2$ et $Y_3$ forment ensemble le groupe de formule $=C=X_1$, ou bien l'un des symboles $Y_1$ et $Y_2$ représente le groupe de formule $-C(=X_1)-Y_4$ dans lequel $Y_4$ est un groupe éliminable, et l'autre et $Y_3$ représentent l'hydrogène, ou leurs sels, ou bien

b) dans un composé de formule IV

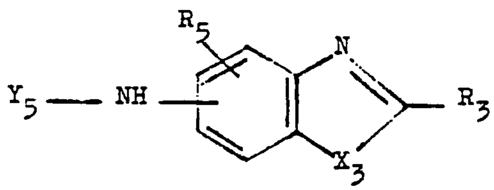

(IV),

dans laquelle $R_5$ représente un groupe convertible en le groupe de formule $R_1-C(=X_1)-$ ou dans un sel d'un tel composé, on convertit $Y_5$ en le groupe de formule $R_1-C(=X_1)-$, ou bien

c) on cyclise un composé de formule

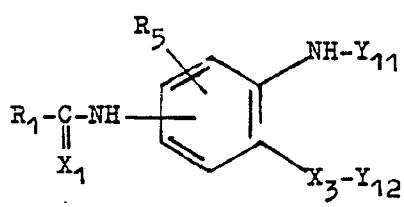

(V),

dans laquelle l'un des symboles $Y_{11}$ et $Y_{12}$ représente le groupe de formule $-C(=Y_{13})-R_3$ et l'autre un groupe éliminable et $Y_{13}$ représente un groupe oxo qui a éventuellement subi une modification fonctionnelle, ou un sel d'un tel composé, et si on le désire, on convertit un composé obtenu comformément à l'invention en un autre composé de formule I et/ou un sel obtenu en le composé libre ou en un autre sel.

36. Les composés qu'on peut obtenir par le procédé de la revendication 35.

37. Un composé selon la revendication 1, pour l'utilisation dans un procédé pour le traitement thérapeutique de l'organisme humain ou animal.

38. Une composition pharmaceutique contenant un composé selon la revendication 1 avec des produits auxiliaires et véhicules pharmaceutiques usuels.